(19) Europäisches Patentamt
European Patent Office
Office européen des brevets

(11) EP 2 414 824 B1

(12) EUROPEAN PATENT SPECIFICATION

(45) Date of publication and mention
of the grant of the patent:
06.12.2017 Bulletin 2017/49

(51) Int Cl.:
G01N 33/48 (2006.01)    G06F 19/00 (2011.01)
C12Q 1/68 (2006.01)

(21) Application number: 10759446.7

(22) Date of filing: 01.04.2010

(86) International application number:
PCT/US2010/029720

(87) International publication number:
WO 2010/115061 (07.10.2010 Gazette 2010/40)

(54) **BIOMARKERS FOR MONITORING TREATMENT OF NEUROPSYCHIATRIC DISEASES**

BIOMARKER ZUR ÜBERWACHUNG DER BEHANDLUNG VON NEUROPSYCHIATRISCHEN ERKRANKUNGEN

BIOMARQUEURS POUR CONTRÔLER LE TRAITEMENT DE MALADIES NEUROPSYCHIATRIQUES

(84) Designated Contracting States:
AT BE BG CH CY CZ DE DK EE ES FI FR GB GR HR HU IE IS IT LI LT LU LV MC MK MT NL NO PL PT RO SE SI SK SM TR

(30) Priority: 01.04.2009 US 165662 P

(43) Date of publication of application:
08.02.2012 Bulletin 2012/06

(73) Proprietor: Vindrauga Holdings, LLC
San Diego, CA 92108 (US)

(72) Inventors:
• PI, Bo
Carlsbad
California 92009 (US)
• BILELLO, John
Durham
North Carolina 27713 (US)

(74) Representative: Grund, Martin
Grund Intellectual Property Group
Patentanwalt und Solicitor PartG mbB
Postfach 44 05 16
80754 München (DE)

(56) References cited:
EP-A1- 1 586 657    WO-A2-02/057790
US-A1- 2008 281 531

• DATABASE WPI Week 200673 Thomson Scientific, London, GB; AN 2006-703206 XP002680727, & KR 2005 0115436 A (KIM E A) 7 December 2005 (2005-12-07)
• IKEDA T ET AL: "Modulation of monoamine transporter expression and function by repetitive transcranial magnetic stimulation", BIOCHEMICAL AND BIOPHYSICAL RESEARCH COMMUNICATIONS, ACADEMIC PRESS INC. ORLANDO, FL, US, vol. 327, no. 1, 4 February 2005 (2005-02-04), pages 218-224, XP004697917, ISSN: 0006-291X, DOI: 10.1016/J.BBRC.2004.12.009

## Description

### CROSS-REFERENCE TO RELATED APPLICATIONS

This application claims benefit of priority from U.S. Provisional Application Serial No. 61/165,662, filed on April 1, 2009.

### TECHNICAL FIELD

This document relates to materials and methods for monitoring the effectiveness of treatment in a subject having neuropsychiatric disease.

### BACKGROUND

Neuropsychiatric diseases include major depression, schizophrenia, mania, post-traumatic stress disorder, Tourette's disorder, Parkinson's disease, and obsessive compulsive disorder. These disorders are often debilitating and difficult to diagnose and treat effectively. Most clinical disorders do not arise due to a single biological change, but rather are the result of interactions between multiple factors. Different individuals affected by the same clinical condition (e.g., major depression) may present with a different range or extent of symptoms, depending on the specific changes within each individual.

WO 02/057790 discloses the measurement of apoptotic cellular marker protein levels and function for the diagnosis of a subject with depression, monitoring of disease state and predicting and monitoring of therapeutic efficiency.

US 2008/0281531 discloses a method of diagnosing whether or not a subject suffers from depression using the peripheral whole blood sample of the subject and measuring the expression level of 18 genes.

EP 1586657 discloses a method of diagnosing the conditions of depression of a patient by analyzing gene expression using mRNA from the peripheral blood to evaluate whether or not the subject is afflicted with depression, the type of depression, and the conditions of depression.

KR 20050115436 discloses identifying the differentially expressed genes in the hippocampus of rat brain affected by repetitive transcranial magnetic stimulation that is used in the therapy of major depression.

Ikeda et al. (Biochem. Biophys. Res. Comm. 2005, 327:218-224) describe a study of mRNA expression changes of monoamine transporter genes which are targets for antidepressants and psychostimulants during a 20 day treatment with repetitive transcranial magnetic stimulation.

### SUMMARY

The invention is defined by the appended claims.

This document is based in part on the development of methods for identifying pharmacodynamic biomarkers of neuropsychiatric disease that can be used for monitoring a subject's response to treatment.

In one aspect, this document features a method for identifying biomarkers of neuropsychiatric disease, comprising (a) calculating a first diagnostic disease score for a subject having said neuropsychiatric disease, wherein said first diagnostic disease score is calculated prior to administration of transcranial magnetic stimulation to said subject; (b)measuring the levels of one or more analytes in a first biological sample obtained from said subject prior to administration of said transcranial magnetic stimulation, wherein the first sample is a blood, serum, or plasma sample; (c) calculating a second diagnostic disease score for said subject after administration of said transcranial magnetic stimulation; (d)measuring the levels of said one or more analytes in a second biological sample obtained from said subject after administration of said transcranial magnetic stimulation, wherein the second biological sample is a bllod, serum, or plasma sample; and (e) identifying one or more analytes as being biomarkers for said neuropsychiatric disease, wherein said one or more analytes are identified as biomarkers if they are differentially expressed between said first and second biological samples, wherein said differential expression of said one or more analytes correlates to a positive or negative change in said subject's diagnostic score.

The neuropsychiatric disease can be major depressive disorder (MDD). The diagnostic scores can be determined by clinical assessment. An analyte can be identified as being a biomarker for the neuropsychiatric disease if the expression level of the analyte is correlated with a positive or negative change in the second diagnostic score relative to the first diagnostic score. The administration of transcranial magnetic stimulation can comprise repetitive transcranial magnetic stimulation. The administration of transcranial magnetic stimulation can comprise stimulating a prefrontal cortex of the subject. The second biological sample can be collected from the subject hours, days, weeks, or months after administering transcranial magnetic stimulation to the subject. Steps (c), (d), and (e) can be repeated at intervals of time after administering transcranial magnetic stimulation to the subject. The subject also can be monitored using molecular imaging technology and/or clinical evaluation tools such as the Hamilton Rating Scale for Depression (HAM-D) Score. The subject can receive one or more additional forms of therapeutic intervention (e.g., one or more additional forms of therapeutic intervention selected from the group consisting of cognitive behavioral therapy, drug therapy, therapeutic interventions that are behavioral in nature, group therapies, interpersonal therapies, psychodynamic therapies, relaxation or meditative therapies, and traditional psychotherapy). In some embodiments, the method can further comprise (f) using biomarker hypermapping technology to identify specific groups of analytes that are differentially expressed between the first and second biological samples, wherein the differential expression of a group of analytes correlates to a positive or negative change in the subject's hyperspace pattern.

This document also features a method for assessing a treatment response to maintain, adjust or stop said treatment in a mammal having major depressive disorder, comprising (a) measuring the levels of inflammatory markers, HPA axis markers, and metabolic markers present in a first biological sample obtained from said mammal prior to administration of said treatment to obtain first numerical values for the levels of said markers, wherein the first biological sample is a blood, serum, or plasma sample, and wherein said inflammatory markers are alpha 1 antitrypsin, alpha 2 macroglobulin, apolipoprotein CIII, and tumor necrosis factor alpha, said HPA axis markers are epidermal growth factor and granulocyte colony stimulating factor, and said metabolic markers are acylation stimulating protein, prolactin, resistin, and testosterone; (b) determining a first diagnostic disease score for the mammal, wherein the first diagnostic disease score is calculated using said first numerical values; (c) measuring the levels of said inflammatory markers, said HPA axis markers, and said metabolic markers present in a second biological sample obtained from said mammal after administration of said treatment to obtain second numerical values for the levels of said markers, wherein the first biological sample is a blood, serum, or plasma sample; (d) determining a second diagnostic disease score for the mammal, wherein the second diagnostic disease score is calculated using said second numerical; and (e) comparing said first diagnostic disease score to said second diagnostic disease score. The method comprises using a hypermap that comprises using a score for said levels of said inflammatory markers, a score for said levels of said HPA axis markers, and a score for said levels of said metabolic markers to compare said first and second diagnostic disease scores. The mammal can be a human. The treatment can be transcranial magnetic stimulation. The first diagnostic disease score can be calculated using numerical values for the levels of said inflammatory markers, said HPA axis markers, said metabolic markers, and at least two neurotrophic markers present in the first and/or biological sample, wherein said at least two neurotrophic markers are selected from the group consisting of brain-derived neurotrophic factor, S100B, neurotrophin 3, reelin, glial cell line derived neurotrophic factor, and artemin.

Unless otherwise defined, all technical and scientific terms used herein have the same meaning as commonly understood by one of ordinary skill in the art to which this disclosure pertains. The materials, methods, and examples are illustrative only and not intended to be limiting.

Other features and advantages of the invention will be apparent from the following detailed description.

## DESCRIPTION OF DRAWINGS

Figure 1 is a flow diagram showing steps that can be taken to identify disease-related biomarkers using defined patient populations and a biomarker library with or without the addition of disease-related content.

Figure 2 is a flow diagram showing steps that can be taken to identify pharmacodynamic biomarkers that indicate a positive or negative response to treatment for a neuropsychiatric disease.

Figure 3 is a biomarker hypermap (BHYPERMAP™) of a dataset used to derive the MDDScore in a study of 50 MDD patients (filled circles) and 20 normal subjects (open circles).

Figure 4 is a biomarker hypermap of changes in patients map positions indicative of a positive or negative response to treatment for a neuropsychiatric disease. Treatment (Rx) was with LEXAPRO™. MDD patients at baseline are indicated by filled circles. Filled triangles represent patients after 2-3 weeks of treatment, and open squares represent patients after 8 weeks of treatment. The open circles represent untreated normal subjects.

Figure 5 is a flow diagram showing steps that can be taken to establish a set of pharmacodynamic biomarkers using mass spectroscopy-based differential protein measurement.

Figure 6 shows an example of a computer-based diagnostic system employing the biomarker analysis described in this document.

Figure 7 shows an example of a computer system that can be used in the computer-based diagnostic system depicted in Figure 6.

## DETAILED DESCRIPTION

The invention is defined by the appended claims.

This document is based in part on the identification of methods for diagnosing depression disorder conditions and monitoring treatment by evaluating (e.g., measuring) biomarker expression. As described herein, this document provides methods and materials for identifying and validating pharmacodynamic biomarkers associated with positive or negative changes in a subject following administration of transcranial magnetic stimulation (TMS). An advantage of using TMS as opposed to antidepressant drugs in assessing physiological changes related to treatment efficacy is that TMS treatment itself is of brief duration and is physical rather than biochemical in nature. The methods and materials provided herein can be used to diagnose patients with neuropsychiatric disorders, determine treatment options, and provide quantitative measurements of treatment efficacy.

*Transcranial Magnetic Stimulation*

This document provides methods for determining a subject's diagnostic scores pre- and post-TMS. TMS is a noninvasive technique used to treat neuropsychiatric diseases such as major depression, schizophrenia, mania, post-traumatic stress disorder, Tourette's disorder, Parkinson's disease, and obsessive compulsive disorder. TMS involves discharging electrical energy through a conducting coil to produce a transient magnetic field that causes an electrical current to flow to a secondary conducting material such as neuronal tissue. Since the scalp and skull are largely nonconductive, the transient magnetic field penetrates these tissues to target specific cortical regions of the brain. Stimulation of the frontal cortex has been demonstrated to induce short- and long-term changes in behavior and mood in healthy subjects and subjects with MDD. For review, see Paus and Barrett, J. Psychiatry Neurosci. 29:268-79 (2004).

A number of methods of administering TMS can be used. An exemplary protocol can be found at neuronetics.com on the World Wide Web. TMS can be administered using either a biphasic or monophasic magnetic pulse. A biphasic pulse is sinusoidal and is generally of shorter duration than a monophasic pulse, which involves a rapid rise from zero followed by a slow decay back to zero. In addition, TMS can be administered using either circular or figure eight-shaped conductive coils. While circular coils are generally more powerful, figure eight-shaped coils produce a more focused magnetic field and a better spatial resolution of activation. An antidepressant effect often is evident at a range (e.g., 1-25 Hz) of frequencies. Both the orientation and intensity of the conductive coil determine the type of tissue stimulated and the strength of that stimulation. In some cases, TMS can be repetitive TMS (rTMS), in which a train of magnetic pulses are administered to a subject. Repetitive TMS using varying frequencies and intensities can increase or decrease excitability in a cortical area directly targeted by the stimulation. For example, the left prefrontal cortex is less active in subjects with clinical depression, and the prefrontal cortex is readily accessible to TMS. Mock stimulation can be used as a control or placebo for TMS or rTMS. The NeuroStar TMS Therapy system (neuronetics.com on the World Wide Web) is an example of an FDA-approved TMS Therapy® device that can be used for treatment of depression and in biomarker studies.

*Diagnostic Score*

This document provides methods and materials for determining a subject's diagnostic score. An exemplary subject for the methods described herein is a human, but subjects also can include animals that are used as models of human disease (e.g., mice, rats, rabbits, dogs, and non-human primates). The methods provided herein can be used to establish a baseline score prior to starting a new therapy regimen or continuing an existing therapy regimen. Diagnostic scores determined post-treatment can be compared to the baseline score in order to observe a positive or negative change relative to baseline. Baseline and post-treatment diagnostic scores can be determined by any suitable method of assessment. For example, in MDD a clinical assessment of the subject's symptoms and well-being can be performed. The "gold standard" diagnostic method is the structured clinical interview. In some cases, a subject's diagnostic score can be determined using the clinically-administered Hamilton Depression Rating Scale (HAMD), a 17-item scale that evaluates depressed mood, vegetative and cognitive symptoms of depression, and co-morbid anxiety symptoms. HAMD can be used to quantify the severity of depressive symptoms at the time of assessment. See Michael Taylor & Max Fink, Melancholia: The Diagnosis, Pathophysiology, and Treatment or Repressive Illness, 91-92, Cambridge University Press (2006). Studies have demonstrated improved HAMD scores following TMS. Other methods of clinical assessment can be used. In some cases, self-rating scales, such as the Beck Depression Inventory scale, can be used. Many rating scales for neuropsychiatric diseases are observer-based. For example, the Montgomery-Åsberg Depression Rating Scale can be used to determine a subject's depression diagnostic score. To determine a diagnostic score based on a subject's overall social, occupational, and psychological functioning, the Global Assessment of Functioning Scale can be used.

In some cases, mathematical algorithms can be used to determine diagnostic scores. Algorithms for determining an individual's disease status or response to treatment, for example, can be determined for any clinical condition. Algorithms for diagnosing or assessing response to treatment, for example, can be determined using metrics (e.g., serum levels of multiple analytes) associated with a defined clinical condition before and/or after treatment. As used herein, an "analyte" is a substance or chemical constituent that can be objectively measured and determined in an analytical procedure such as, without limitation, immunoassay or mass spectrometry. The algorithms discussed herein can be mathematic functions containing multiple parameters that can be quantified using, for example, medical devices, clinical assessment scores, or biological or physiological analysis of biological samples. Each mathematic function can be a weight-adjusted expression of the levels of parameters determined to be relevant to a selected clinical condition. Algorithms generally can be expressed in the format of Formula 1:

$$\text{Diagnostic score} = f(x1, x2, x3, x4, x5, \ldots xn) \qquad (1)$$

The diagnostic score is a value that is the diagnostic or prognostic result, "f" is any mathematical function, "n" is any integer (e.g., an integer from 1 to 10,000), and x1, x2, x3, x4, x5 ... xn are the "n" parameters that are, for example, measurements determined by medical devices, clinical assessment scores, and/or test results for biological samples (e.g., human biological samples such as blood, serum, plasma, urine, or cerebrospinal fluid).

Parameters of an algorithm can be individually weighted. An example of such an algorithm is expressed in Formula 2:

$$\text{Diagnostic score} = a1*x1 + a2*x2 - a3*x3 + a4*x4 - a5*x5 \qquad (2)$$

Here, x1, x2, x3, x4, and x5 are measurements determined by medical devices, clinical assessment scores, and/or test results for biological samples, and a1, a2, a3, a4, and a5 are weight-adjusted factors for x1, x2, x3, x4, and x5, respectively.

A diagnostic score can be used to quantitatively define a medical condition or disease, or the effect of a medical treatment. For example, a computer can be used to populate an algorithm, which then can be used to determine a diagnostic score for a disorder such as depression. In such an embodiment, the degree of depression can be defined based on Formula 1, with the following general formula:

$$\text{Depression diagnosis score} = f(x1, x2, x3, x4, x5 \ldots xn)$$

The depression diagnosis score is a quantitative number that can be used to measure the status or severity of depression in an individual, "f" is any mathematical function, "n" can be any integer (e.g., an integer from 1 to 10,000), and x1, x2, x3, x4, x5 ... xn are, for example, the "n" parameters that are measurements determined using medical devices, clinical evaluation scores, and/or test results for biological samples (e.g., human biological samples).

In a more general form, multiple diagnostic scores Sm can be generated by applying multiple formulas to specific groupings of biomarker measurements, as illustrated in Formula 3:

$$\text{Diagnostic scores } Sm = Fm(x1, \ldots xn) \qquad (3)$$

Multiple scores can be useful, for example, in the identification of specific types and subtypes of depressive disorders and/or associated disorders. In some cases, the depressive disorder is major depressive disorder (MDD). Multiple scores can also be parameters indicating patient treatment progress or the efficacy of the treatment selected. Diagnostic scores for subtypes of depressive disorders can aid in the selection or optimization of antidepressants or other pharmaceuticals.

Biomarker expression level changes can be expressed in the format of Formula 4:

$$C_{mi} = M_{ib} - M_{ia} \qquad (4)$$

where $M_{ib}$ and $M_{ia}$ are expression levels of a biomarker before and after treatment, respectively. Change in a subject's diagnostic score can be expressed in the format of Formula 5:

$$H = HAMD_b - HAMD_a \qquad (5)$$

where $HAMD_b$ and $HAMD_a$ are diagnostic scores before and after treatment, respectively. A pre-established process can be used to select only subjects having a $HAMD_a$ score greater than a minimum cut-off value (Eh = efficacy cut-off value). Upon statistical evaluation, where statistical significance is defined as $p < 0.05$, a biomarker having a p value less than 0.05 can be selected as a biomarker associated with therapy-responsive MDD.

*Use of Biomarker Hypermapping*

This document also provides methods for using biomarker hypermapping to evaluate patients pre- and post-TMS. This approach uniquely includes the construction of a multianalyte hypermap versus analyzing single markers either alone or in groups. Biomarker hypermapping uses multiple markers from a human biomarker collection and interrelated algorithms to distinguish individual groups of patients. Using clusters of biomarkers reflective of different physiologic parameters (e.g., hormones vs. inflammatory markers), a patient's biomarker responses can be mapped onto a multi-dimensional hyperspace. As described herein, four classes of biomarkers are used in the process of mapping changes in response to therapy:

> Inflammatory biomarkers
> HPA axis biomarkers
> Metabolic biomarkers
> Neurotrophic biomarkers

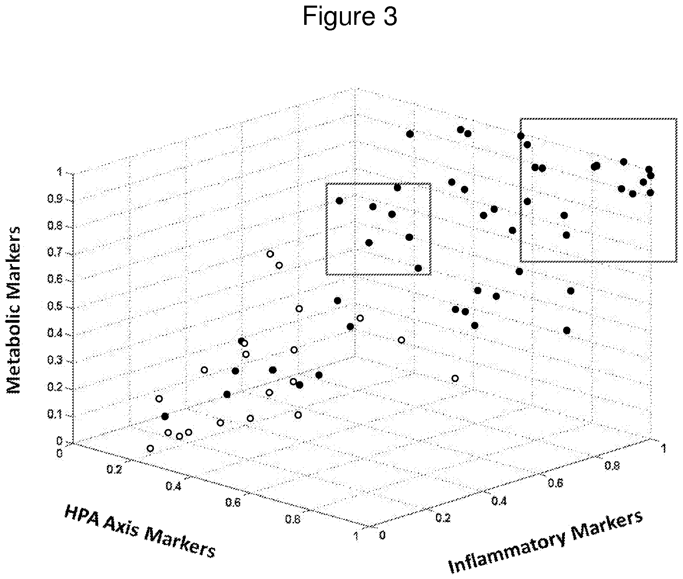

Four vectors can be created for the four classes of biomarkers; together, the vectors form a point in a hyperspace. A computer program can be used to analyze the data, plot the vectors, and populate the hypermap. For ease of visualization, a three-dimensional hypermap can be created using vectors established from three of the four classes of physiologically defined biomarkers. This initially can be done for a patient at the time s/he is first tested, to aid in their classification. Figure 3 illustrates the concept. Distinct coefficients were used to create hyperspace vectors for 50 MDD patients and 20 age-matched normal subjects. Multiplex biomarker data from clinical samples were used to display individual patients (filled circles) and normal subjects (open circles) on a hyperspace map where the axes are HPA axis, inflammatory and metabolic markers. Unlike the MDD score that provides a numerical value for the patient, the hypermap discloses information relative to the expression of different classes of markers. By way of example, the patients in the small square have higher values for metabolic and inflammatory markers, while those in the larger rectangle have high values for HPA axis markers in addition to the two other marker groups. As clinically relevant information (e.g., disease severity) is collected on increasingly larger numbers of patients, this technology may be an even more potent aid to patient management.

Further, a hypermap can, by addition of data on patient response, answer questions about preferred treatment regimens and assessment of treatment efficacy. By way of example, using a hypermap that incorporates a large amount of patient data surrounding biomarker changes and clinical response to a selective serotonin reuptake inhibitor (SSRI), areas of hyperspace (patterns) associated with an enhanced response to TMS vs. LEXAPRO3 [a serotonin and norepinephrin reuptake inhibitor (SNRI)] can be identified.

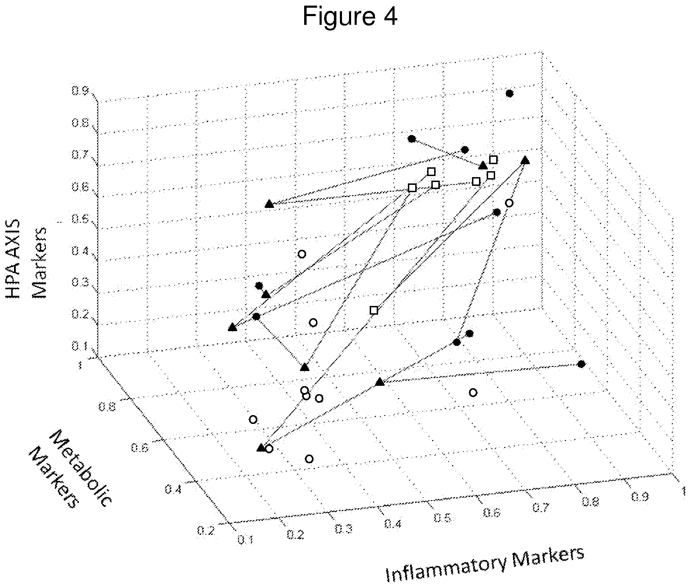

Figure 4 shows a specific example of a biomarker hypermap indicating positive or negative response to treatment for a series of patients treated with LEXAPRO3. MDD patients at baseline are indicated by filled circles. Filled triangles represent patients after 2-3 weeks of treatment, and open squares represent patients after 8 weeks of treatment. Open circles represent untreated normal subjects.

*Identifying Biomarkers Associated with Neuropsychiatric Disease and Therapy*

This document provides methods for identifying treatment-responsive biomarkers. As used herein, a "biomarker" is a characteristic that can be objectively measured and evaluated as an indicator of a normal biologic or pathogenic process or pharmacological response to a therapeutic intervention. Biomarker panels and their associated algorithms can encompass one or more analytes (e.g., proteins, nucleic acids, and metabolites), physical measurements, or combinations thereof.

As used herein, a "pharmacodynamic" biomarker is a biomarker that can be used to quantitatively evaluate (e.g., measure) the impact of treatment or therapeutic intervention on the course, severity, status, symptomology, or resolution of a disease. In some embodiments, pharmacodynamic biomarkers can be identified based on a correlation or the defined relationship between analyte expression levels and positive or negative changes in a subject's diagnostic score (e.g., HAMD score in depression) relative to one or more pre-treatment baseline scores. In some cases, analyte expression levels can be measured in samples collected from a subject prior to and following TMS or mock stimulation. Analyte expression levels in the pre-TMS sample can be compared to analyte levels in the post-TMS samples. If the change in expression corresponds to positive or negative clinical outcomes, as determined by an improvement in the post-TMS diagnostic score relative to the pre-TMS diagnostic score, the analyte can be identified as pharmacodynamic biomarker for MDD and other neuropsychiatric diseases.

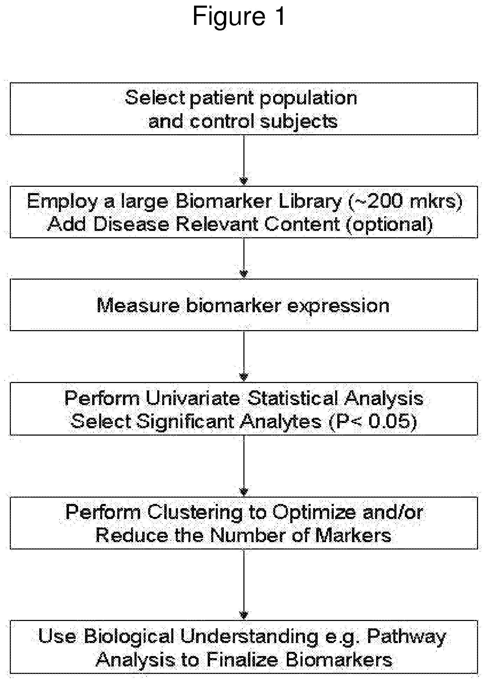

Pharmacodynamic biomarkers identified by the methods and materials provided herein can be previously unknown factors or biomolecules known to be associated with neuropsychiatric diseases. A procedure for using a biomarker library to identify potential neuropsychiatric biomarkers is diagrammed in Figure 1. As a starting point, a library can include analytes generally indicative of inflammation, cellular adhesion, immune responses, or tissue remodeling. In some embodiments (e.g., during initial library development), a library may include a dozen or more markers, a hundred markers, or several hundred markers. For example, a biomarker library can include a few hundred (e.g., about 200, about 250, about 300, about 350, about 400, about 450, or about 500) protein analytes. New markers can be added, such as markers specific to individual disease states, and/or markers that are more generalized, such as growth factors. A biomarker library can be refined by identification of disease-related proteins obtained from discovery research (e.g., using differential display techniques, such as isotope coded affinity tags (ICAT), accurate mass and time tags or other mass spectroscopy techniques). In this manner, a library can become increasingly specific to a particular disease state.

Many biomolecules are either up-regulated or down-regulated in subjects having different neuropsychiatric diseases. Numerous transcription factors, growth factors, hormones, and other biological molecules are associated with neuropsychiatric diseases. The parameters used to define biomarkers for MDD and other neuropsychiatric diseases can be selected from, for example, the functional groupings consisting of inflammatory biomarkers, HPA axis factors, metabolic biomarkers, and neurotrophic factors, including neurotrophins, glial cell-line derived neurotrophic factor family ligands (GFLs), and neuropoietic cytokines. Biomarkers of neuropsychiatric disease can be, for example, factors involved in the inflammatory response. A wide variety of proteins are involved in inflammation, and any one of them is open to a genetic mutation that impairs or otherwise disrupts the normal expression and function of that protein. Inflammation also induces high systemic levels of acute-phase proteins. These proteins include C-reactive protein, serum amyloid A, serum amyloid P, vasopressin, and glucocorticoids, which cause a range of systemic effects. Inflammation also involves release of proinflammatory cytokines and chemokines. Studies have demonstrated that abnormal functioning of the inflammatory response system disrupts feedback regulation of the immune system, thereby contributing to the development of neuropsychiatric and immunologic disorders. In fact, several medical illnesses that are characterized by chronic inflammatory responses (e.g., rheumatoid arthritis) have been reported to be accompanied by depression. Furthermore, recent evidence has linked elevated levels of inflammatory cytokines with both depression and cachexia, and experiments have shown that introducing cytokines induces depression and cachectic symptoms in both humans and rodents, suggesting that there may be a common etiology at the molecular level. For example, administration of proinflammatory cytokines (e.g., in cancer or hepatitis C therapies) can induce "sickness behavior" in animals, which is a pattern of behavioral alterations that is very similar to the behavioral symptoms of depression in humans. Therapeutic agents targeting specific cytokine molecules, such as tumor necrosis factor-alpha, are currently being evaluated for their potential to simultaneously treat both depression and cachexia pharmacologically. In sum, the "Inflammatory Response System (IRS) model of depression" (Maes, Adv. Exp. Med. Biol. 461:25-46 (1999)) proposes that proinflammatory cytokines, acting as neuromodulators, represent key factors in mediation of the behavioral, neuroendocrine and neurochemical features of depressive disorders.

In some cases, neuropsychiatric disease biomarkers can be neurotrophic factors. Most neurotrophic factors belong to one of three families: (1) neurotrophins, (2) glial cell-line derived neurotrophic factor family ligands (GFLs), and (3) neuropoietic cytokines. Each family has its own distinct signaling family, yet the cellular responses elicited often overlap. Neurotrophic factors such as brain-derived neurotrophic factor (BDNF) and its receptor, TrkB, are proteins responsible for the growth and survival of developing neurons and for the maintenance of mature neurons. Neurotrophic factors can promote the initial growth and development of neurons in the CNS and PNS, as well as regrowth of damaged neurons *in vitro* and *in vivo.* Neurotrophic factors often are released by a target tissue in order to guide the growth of developing axons. Studies have suggested that deficits in neurotrophic factor synthesis may be responsible for increased apoptosis in the hippocampus and prefrontal cortex that is associated with the cognitive impairment described in depression.

In some cases, neuropsychiatric biomarkers can be factors of the HPA axis. The HPA axis, also known as the limbic-hypothalamic-pituitary-adrenal axis (LHPA axis), is a complex set of direct influences and feedback interactions among the hypothalamus (a hollow, funnel-shaped part of the brain), the pituitary gland (a pea-shaped structure located below the hypothalamus), and the adrenal (or suprarenal) glands (small, conical organs on top of the kidneys). Interactions among these organs constitute the HPA axis, a major part of the neuroendocrine system that controls the body's stress response and regulates digestion, the immune system, mood, and energy storage and expenditure. The HPA axis is dysregulated in several psychiatric and neuropsychiatric diseases, as well as in alcoholism and stroke. Examples of HPA axis biomarkers include ACTH and cortisol. Cortisol inhibits secretion of corticotropin-releasing hormone (CRH), resulting in feedback inhibition of ACTH secretion. This normal feedback loop may break down when humans are exposed to chronic stress, and may be an underlying cause of depression.

In some cases, metabolic factors can be useful biomarkers for neuropsychiatric disease. Metabolic biomarkers are a set of biomarkers that provide insight into metabolic processes in wellness and disease states. Human diseases manifest in complex downstream effects, affecting multiple biochemical pathways. For example, depression and other neuropsychiatric diseases often are associated with metabolic disorders such as diabetes. Consequently, various metabolites and the proteins and hormones controlling metabolic processes can be used for diagnosing depressive disorders such as MDD, stratifying disease severity, and monitoring a subject's response to treatment for the depressive disorder.

Table 1 provides an exemplary list of inflammatory biomarkers.

**Table 1**

| Gene Symbol | Gene Name | Cluster |
|---|---|---|
| A1AT | Alpha 1 Antitrypsin | Inflammation |
| A2M | Alpha 2 Macroglobin | Inflammation |
| AGP | Alpha 1-Acid Glycoprotein | Inflammation |
| ApoC3 | Apolipoprotein CIII | Inflammation |
| CD40L | CD40 ligand | Inflammation |
| IL-1($\alpha$ or $\beta$) | Interleukin 1 | Inflammation |
| IL-6 | Interleukin 6 | Inflammation |
| IL-13 | Interleukin 13 | Inflammation |
| IL-18 | Interleukin 18 | Inflammation |
| IL-1ra | Interleukin 1 Receptor Antagonist | Inflammation |
| MPO | Myeloperoxidase | Inflammation |
| PAI-1 | Plasminogen activator inhibitor-1 | Inflammation |
| RANTES | RANTES (CCL5) | Inflammation |
| TNFA | Tumor Necrosis Factor alpha | Inflammation |
| STNFR | Soluble TNFIreceptor (I,II) | Inflammation |

Table 2 provides and exemplary list of HPA axis biomarkers.

**Table 2**

| Gene Symbol | Gene Name | Cluster |
|---|---|---|
| None | Cortisol | HPA axis |
| EGF | Epidermal Growth Factor | HPA axis |
| GCSF | Granulocyte Colony Stimulating Factor | HPA axis |
| PPY | Pancreatic Polypeptide | HPA axis |
| ACTH | Adrenocorticotropic hormone | HPA axis |
| AVP | Arginine Vasopressin | HPA axis |
| CRH | Corticotropin-Releasing Hormone | HPA axis |

Table 3 provides an exemplary list of metabolic biomarkers.

**Table 3**

| Gene Symbol | Gene Name | Cluster |
|---|---|---|
| ACRP30 | Adiponectin | Metabolic |
| ASP | Acylation Stimulating Protein | Metabolic |
| FABP | Fatty Acid Binding Protein | Metabolic |

(continued)

| Gene Symbol | Gene Name | Cluster |
|---|---|---|
| INS | Insulin | Metabolic |
| LEP | Leptin | Metabolic |
| PRL | Prolactin | Metabolic |
| RETN | Resistin | Metabolic |
| None | Testosterone | Metabolic |
| TSH | Thyroid Stimulating Hormone | Metabolic |
| None | Thyroxine | Metabolic |

Table 4 provides an exemplary list of neurotrophic biomarkers.

**Table 4**

| Gene Symbol | Gene Name | Cluster |
|---|---|---|
| BDNF | Brain-derived neurotrophic factor | Neurotrophic |
| S100B | S100B | Neurotrophic |
| NTF3 | Neurotrophin 3 | Neurotrophic |
| RELN | Reelin | Neurotrophic |
| GDNF | Glial cell line derived neurotrophic factor | Neurotrophic |
| ARTN | Artemin | Neurotrophic |

*Qualifying Biomarkers*

This document also provides materials and methods for qualifying both disease related and pharmacodynamic biomarkers. At present there is no consistent framework for acceptance and qualification of biomarkers for regulatory use. Such a framework is needed to facilitate innovative and efficient research and subsequent application of biomarkers in drug and therapeutic regimen development. Furthermore, there currently is no evidentiary process that is fully acceptable to the Food and Drug Administration. Nevertheless, it is apparent that cumulative data from multiple laboratories (perhaps a biomarker consortium model) will drive efficient execution of research and ultimately regulatory acceptance of biomarkers for specific indications. In the assessment of complex diseases including neuropsychiatric diseases such as MDD, as described herein, studies of well characterized patient and control normal subjects have been undertaken as part of a biomarker qualification process. Biomarker qualification is a graded, "fit-for-purpose" evidentiary process that links a biomarker with biology and with clinical end points. As clinical experience with biomarker panels is developed, information relevant to biomarker qualification and eventually regulatory acceptance of biomarkers also is developed for specific disease applications, as well as pharmacodynamic and efficacy markers.

Traditional cumulative clinical studies (e.g., assaying biological samples, clinical measures, imaging analysis) can be used in the qualification process. In some cases, biomarker expression can be measured in a statistically powered cohort of patients treated by TMS or placebo (i.e., without magnetic pulse). The age and sex of the cohort of patients can be adjusted to conform to the distribution of MDD patients in the general population. Such studies can reveal the possibility and nature of a placebo effect in TMS therapy. In the case of MDD, comparisons can be made between biomarkers with a TMS-positive response to positive changes observed in patients being treated with antidepressant pharmaceuticals, electro-convulsive treatment (ECT), or cognitive behavioral therapy (CBT).

*Analyte Measurement and Algorithm Calculation*

A number of methods can be used to quantify treatment-specific analyte expression. For example, measurements can be obtained using one or more medical devices or clinical evaluation scores to assess a subject's condition, or using tests of biological samples to determine the levels of particular analytes. As used herein, a "biological sample" is a sample that contains cells or cellular material, from which nucleic acids, polypeptides, or other analytes can be obtained. Depending upon the type of analysis being performed, a biological sample can be serum, plasma, or blood cells isolated by standard techniques. Serum and plasma are exemplary biological samples, but other biological samples can be used. For example, specific monoamines can be measured in urine, and depressed patients as a group have been found to excrete greater amounts of catecholamines (CAs) and metabolites in urine than healthy control subjects. Examples of other suitable biological samples include, without limitation, cerebrospinal fluid, pleural fluid, bronchial lavages, sputum, peritoneal fluid, bladder washings, secretions (e.g., breast secretions), oral washings, swabs (e.g., oral swabs), isolated cells, tissue samples, touch preps, and fine-needle aspirates. In some cases, if a biological sample is to be tested immediately, the sample can be maintained at room temperature; otherwise the sample can be refrigerated or frozen (e.g., at -80°C) prior to assay. In some cases, samples are collected from the subject at regular intervals following TMS or mock stimulation. In some cases, samples can be collected minutes, hours, days, or weeks following TMS or mock stimulation.

Multiplex methods of quantifying biomarkers are particularly useful. An example of platform useful for multiplexing is the FDA approved, flow-based Luminex assay system (xMAP; online at luminexcorp.com), which permits multiplexing of up to 100 unique assays within a single sample. This multiplex technology uses flow cytometry to detect antibody/peptide/oligonucleotide or receptor tagged and labeled microspheres. Since the system is open in architecture, Luminex can be readily adapted to host particular disease panels.

Another useful technique for analyte quantification is immunoassay, a biochemical test that measures the concentration of a substance (e.g., in a biological tissue or fluid such as serum, plasma, cerebral spinal fluid, or urine) based on the specific binding of an antibody to its antigen. Antibodies chosen for biomarker quantification must have a high affinity for their antigens. A vast array of different labels and assay strategies has been developed to meet the requirements of quantifying plasma proteins with sensitivity, accuracy, reliability, and convenience. For example, Enzyme Linked ImmunoSorbant Assay (ELISA) can be used to quantify biomarkers a biological sample. In a "solid phase sandwich ELISA," an unknown amount of a specific "capture" antibody can be affixed to a surface of a multiwell plate, and the sample can be allowed to absorb to the capture antibody. A second specific, labeled antibody then can be washed over the surface so that it can bind to the antigen. The second antibody is linked to an enzyme, and in the final step a substance is added that can be converted by the enzyme to generate a detectable signal (e.g., a fluorescent signal). For fluorescence ELISA, a plate reader can be used to measure the signal produced when light of the appropriate wavelength is shown upon the sample. The quantification of the assays endpoint involves reading the absorbance of the colored solution in different wells on the multiwell plate. A range of plate readers are available that incorporate a spectrophotometer to allow precise measurement of the colored solution. Some automated systems, such as the BIOMEK® 1000 (Beckman Instruments, Inc.; Fullerton, CA), also have built-in detection systems. In general, a computer can be used to fit the unknown data points to experimentally derived concentration curves.

In some cases, analyte expression levels in a biological sample can be measured using a mass spectrometry instrument (e.g., a multi-isotope imaging mass spectrometry (MIMS) instrument), or any other suitable technology, including for example, technology for measuring expression of RNA. Such methods include, for example, PCR and quantitative real time PCR methods using a dual-labeled fluorogenic probe (e.g., TAQMAN™, Applied Biosystems, Foster City, CA). In some cases, DNA microarrays can be used to study gene expression patterns on a genomic scale. Microarrays allow for simultaneous measurement of changes in the levels of thousands of messenger RNAs within a single experiment. Microarrays can be used to assay gene expression across a large portion of the genome prior to, during, and/or after a treatment regimen. The combination of microarrays and bioinformatics can be used to identify biomolecules that are correlated to a particular treatment regimen or to a positive or negative response to treatment. In some cases, microarrays can be used in conjunction with proteomic analysis.

Useful platforms for simultaneously quantifying multiple protein parameters include, for example, those described in U.S. Provisional Application Nos. 60/910,217 and 60/824,471, U.S. Utility Application No. 11/850,550, and PCT Publication No. WO2007/067819. An example of a useful platform utilizes MIMS label-free assay technology developed by Precision Human Biolaboratories, Inc. (now Ridge Diagnostics, Inc., Research Triangle Park, N.C.). Briefly, local interference at the boundary of a thin film can be the basis for optical detection technologies. For biomolecular interaction analysis, glass chips with an interference layer of $SiO_2$ can be used as a sensor. Molecules binding at the surface of this layer increase the optical thickness of the interference film, which can be determined as set forth in U.S. Provisional Application Nos. 60/910,217 and 60/824,471, for example.

With regard to the potential for new biomarker discovery, traditional 2-dimensional gel electrophoresis can be performed for protein separation, followed by mass spectrometry (e.g., MALDI-TOF, MALDI-ESI) and bioinformatics for protein identification and characterization. Other methods of differential protein quantification can be used. For example, tandem mass spectrometry (MS/MS) can be used to simultaneously determine both the identity and relative abundances of proteins and peptides.

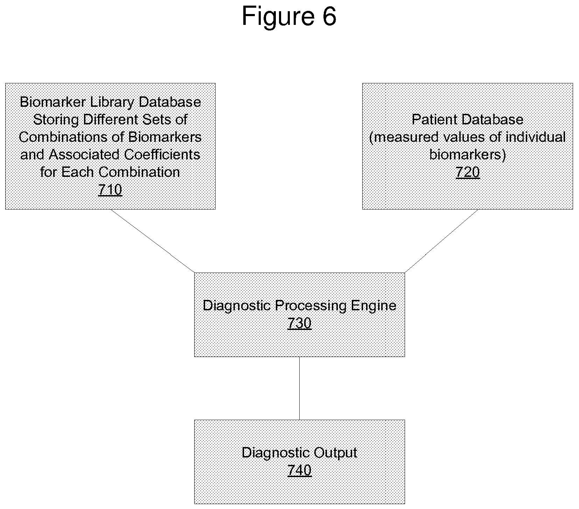

Figure 6 shows an example of a computer-based diagnostic system employing the biomarker analysis described herein. This system includes a biomarker library database 710 that stores different sets combinations of biomarkers and associated coefficients for each combination based on biomarker algorithms which are generated based on, e.g., the methods described herein. The database 710 is stored in a digital storage device in the system. A patient database 720 is provided in this system to store measured values of individual biomarkers of one or more patients under analysis. A diagnostic processing engine 730, which can be implemented by one or more computer processors, is provided to apply one or more sets of combinations of biomarkers in the biomarker library database 710 to the patient data of a particular patient stored in the database 720 to generate diagnostic output for a set of combination of biomarkers that is selected for diagnosing the patient. Two or more such sets may be applied to the patient data to provide two or more different diagnostic output results. The output of the processing engine 730 can be stored in an output device 740, which can be, e.g., a display device, a printer, or a database.

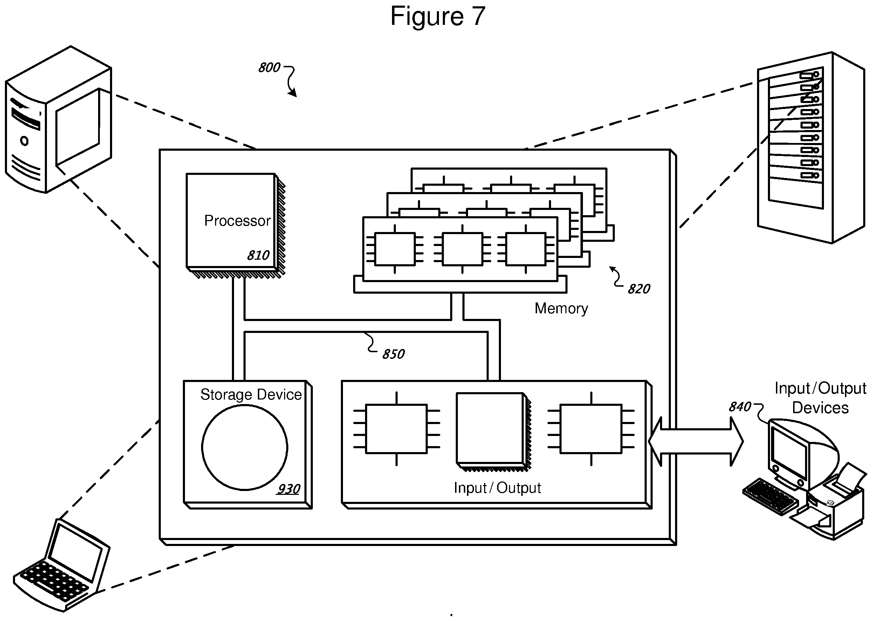

One or more computer systems can be used to implement the system in Figure 6 and for the operations described in association with any of the computer-implement methods described in this document. Figure 7 shows an example of such a computer system 800. The system 800 can include various forms of digital computers, such as laptops, desktops, workstations, personal digital assistants, servers, blade servers, mainframes, and other appropriate computers. The system 800 can also include mobile devices, such as personal digital assistants, cellular telephones, smartphones, and other similar computing devices. Additionally the system can include portable storage media, such as, Universal Serial Bus (USB) flash drives. For example, the USB flash drives may store operating systems and other applications. The USB flash drives can include input/output components, such as a wireless transmitter or USB connector that may be inserted into a USB port of another computing device.

In the specific example in Figure 7, the system 800 includes a processor 810, a memory 820, a storage device 830, and an input/output device 840. Each of the components 810, 820, 830, and 840 are interconnected using a system bus 850. The processor 810 is capable of processing instructions for execution within the system 800. The processor may be designed using any of a number of architectures. For example, the processor 810 may be a CISC (Complex Instruction Set Computers) processor, a RISC (Reduced Instruction Set Computer) processor, or a MISC (Minimal Instruction Set Computer) processor.

In some embodiments, the processor 810 is a single-threaded processor. In other embodiments, the processor 810 is a multi-threaded processor. The processor 810 is capable of processing instructions stored in the memory 820 or on the storage device 830 to display graphical information for a user interface on the input/output device 840.

The memory 820 stores information within the system 800. In some embodiments, the memory 820 is a computer-readable medium. In other embodiments, the memory 820 is a volatile memory unit. In still other embodiments, the memory 820 is a non-volatile memory unit.

The storage device 830 is capable of providing mass storage for the system 800. In some embodiments, the storage device 830 is a computer-readable medium. In various different embodiments, the storage device 830 may be a floppy disk device, a hard disk device, an optical disk device, or a tape device.

The input/output device 840 provides input/output operations for the system 800. In some embodiments, the input/output device 840 includes a keyboard and/or pointing device. In some cases, the input/output device 840 includes a display unit for displaying graphical user interfaces.

The features described can be implemented in digital electronic circuitry, or in computer hardware, firmware, software, or in combinations of them. The apparatus can be implemented in a computer program product tangibly embodied in an information carrier, e.g., in a machine-readable storage device for execution by a programmable processor; and method steps can be performed by a programmable processor executing a program of instructions to perform functions of the described implementations by operating on input data and generating output. The described features can be implemented advantageously in one or more computer programs that are executable on a programmable system including at least one programmable processor coupled to receive data and instructions from, and to transmit data and instructions to, a data storage system, at least one input device, and at least one output device. A computer program is a set of instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

Suitable processors for the execution of a program of instructions include, by way of example, both general and special purpose microprocessors, and the sole processor or one of multiple processors of any kind of computer. Generally, a processor will receive instructions and data from a read-only memory or a random access memory or both. The essential elements of a computer are a processor for executing instructions and one or more memories for storing instructions and data. Generally, a computer will also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including by way of example semiconductor memory devices, such as EPROM, EEPROM, and flash memory devices; magnetic disks such as internal hard disks and removable disks; magneto-optical disks; and CD-ROM and DVD-ROM disks. The processor and the memory can be supplemented by, or incorporated in, ASICs (application-specific integrated circuits).

To provide for interaction with a user, the features can be implemented on a computer having a display device such as a CRT (cathode ray tube) or LCD (liquid crystal display) monitor for displaying information to the user and a keyboard and a pointing device such as a mouse or a trackball by which the user can provide input to the computer.

The features can be implemented in a computer system that includes a back-end component, such as a data server, or that includes a middleware component, such as an application server or an Internet server, or that includes a front-end component, such as a client computer having a graphical user interface or an Internet browser, or any combination of them. The components of the system can be connected by any form or medium of digital data communication such as a communication network. Examples of communication networks include a local area network ("LAN"), a wide area network ("WAN"), peer-to-peer networks (having ad-hoc or static members), grid computing infrastructures, and the Internet.

The computer system can include clients and servers. A client and server are generally remote from each other and typically interact through a network, such as the described one. The relationship of client and server arises by virtue of computer programs running on the respective computers and having a client-server relationship to each other.

*Methods for Using Biomarker Information*

Diagnostic scores and pharmacodynamic biomarkers can be used for, without limitation, treatment monitoring. For example, diagnostic scores and/or biomarker levels can be provided to a clinician for use in establishing or altering a course of treatment for a subject. When a treatment is selected and treatment starts, the subject can be monitored periodically by collecting biological samples at two or more intervals, determining a diagnostic score corresponding to a given time interval pre- and post-treatment, and comparing diagnostic scores over time. On the basis of these scores and any trends observed with respect to increasing, decreasing, or stabilizing diagnostic scores or changes in pharmacodynamic biomarker levels, a clinician, therapist, or other health-care professional may choose to continue treatment as is, to discontinue treatment, or to adjust the treatment plan with the goal of seeing improvement over time. For example, an increase in the level of a pharmacodynamic biomarker that correlates to positive responses to a particular treatment regimen for neuropsychiatric disease can indicate a patient's positive response to treatment. A decrease in the level of such a pharmacodynamic biomarker can indicate failure to respond positively to treatment and/or the need to reevaluate the current treatment plan. Stasis with respect to biomarker expression levels and diagnostic scores can correspond to stasis with respect to symptoms of a neuropsychiatric disease. The biomarker pattern may be different for patients who are on antidepressants or are undergoing other forms of therapy (e.g., cognitive behavioral or electro-convulsive therapy) in addition to TMS, and changes in the diagnostic score toward that of normal patients can be an indication of an effective therapy combination. As the cumulative experience with therapies increases, specific biomarker panels can be derived to monitor responses to TMS in combination with therapy with specific antidepressants, etc.

After a patient's diagnostic scores are reported, a health-care professional can take one or more actions that can affect patient care. For example, a health-care professional can record the diagnostic scores and biomarker expression levels in a patient's medical record. In some cases, a health-care professional can record a diagnosis of a neuropsychiatric disease, or otherwise transform the patient's medical record, to reflect the patient's medical condition. In some cases, a health-care professional can review and evaluate a patient's medical record, and can assess multiple treatment strategies for clinical intervention of a patient's condition.

For major depressive disorder and other mood disorders, treatment monitoring can help a clinician adjust treatment dose(s) and duration. An indication of a subset of alterations in individual biomarker levels that more closely resemble normal homeostasis can assist a clinician in assessing the efficacy of a regimen. A health-care professional can initiate or modify treatment for symptoms of depression and other neuropsychiatric diseases after receiving information regarding a patient's diagnostic score. In some cases, previous reports of diagnostic scores and/or biomarker levels can be compared with recently communicated diagnostic scores and/or disease states. On the basis of such comparison, a health-care profession may recommend a change in therapy. In some cases, a health-care professional can enroll a patient in a clinical trial for novel therapeutic intervention of MDD symptoms. In some cases, a health-care professional can elect waiting to begin therapy until the patient's symptoms require clinical intervention.

A health-care professional can communicate diagnostic scores and/or biomarker levels to a patient or a patient's family. In some cases, a health-care professional can provide a patient and/or a patient's family with information regarding MDD, including treatment options, prognosis, and referrals to specialists, e.g., neurologists and/or counselors. In some cases, a health-care professional can provide a copy of a patient's medical records to communicate diagnostic scores and/or disease states to a specialist.

A research professional can apply information regarding a subject's diagnostic scores and/or biomarker levels to advance MDD research. For example, a researcher can compile data on diagnostic scores with information regarding the efficacy of a drug for treatment of depression symptoms, or the symptoms of other neuropsychiatric diseases, to identify an effective treatment. In some cases, a research professional can obtain a subject's diagnostic scores and/or biomarker levels to evaluate a subject's enrollment or continued participation in a research study or clinical trial. In some cases, a research professional can communicate a subject's diagnostic scores and/or biomarker levels to a health-care professional, and/or can refer a subject to a health-care professional for clinical assessment and treatment of neuropsychiatric disease.

Any appropriate method can be used to communicate information to another person (e.g., a professional), and information can be communicated directly or indirectly. For example, a laboratory technician can input diagnostic scores and/or individual analyte levels into a computer-based record. In some cases, information can be communicated by making a physical alteration to medical or research records. For example, a medical professional can make a permanent notation or flag a medical record for communicating a diagnosis to other health-care professionals reviewing the record. Any type of communication can be used (e.g., mail, e-mail, telephone, facsimile and face-to-face interactions). Secure types of communication (e.g., facsimile, mail, and face-to-face interactions) can be particularly useful. Information also can be communicated to a professional by making that information electronically available (e.g., in a secure manner) to the professional. For example, information can be placed on a computer database such that a health-care professional can access the information. In addition, information can be communicated to a hospital, clinic, or research facility serving as an agent for the professional. The Health Insurance Portability and Accountability Act (HIPAA) requires information systems housing patient health information to be protected from intrusion. Thus, information transferred over open networks (e.g., the internet or e-mail) can be encrypted. When closed systems or networks are used, existing access controls can be sufficient.

The following examples provide additional information on various features described above.

## EXAMPLES

Examples not falling within the scope of the claims are for illustrative purposes only.

Example 1 - Identification of Pharmacodynamic Biomarkers Associated with MDD

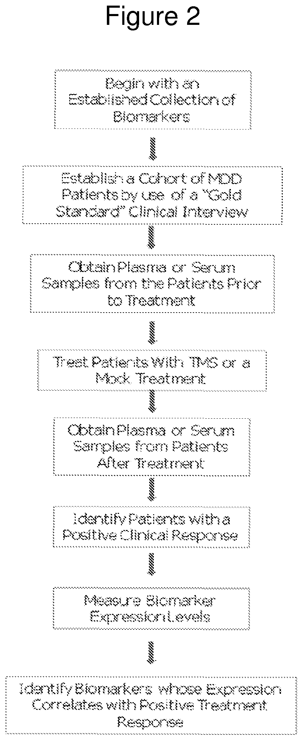

Figure 2 illustrates a process of identifying pharmacodynamic biomarkers for MDD. A collection of biomarkers that have a potential association with MDD is selected based on the result of earlier studies, from a literature search, from genomic or proteomic analysis of biological pathways, or from molecular imaging studies. A cohort of MDD patients are identified using a "gold standard" method of interview-based clinical assessment. Plasma or serum samples are collected from each patient. Patients are then subjected to transcranial magnetic stimulation or mock stimulation (placebo). Post-treatment plasma or serum samples are collected from each patient over a period of time (e.g., minutes, hours, days, and/or weeks after treatment). Expression levels of the selected biomarkers are measured for each sample. The patient's response to treatment, as determined by conducting additional structured clinical interviews and assigning post-TMS diagnostic scores, is recorded. Patients demonstrating a positive clinical response to TMS, which is defined as an improved post-treatment diagnostic score relative to the pre-treatment baseline score, are identified. Analytes whose expression correlates with positive clinical outcomes are identified as pharmacodynamic biomarkers for MDD.

Diagnostic biomarkers for MDD were generated using the steps outlined in Figure 1, and a panel of about 20 analytes was established. These analytes included alpha-2-macroglobin (A2M), brain-derived neurotrophic factor (BDNF), C-reactive protein (CRP), cortisol, epidermal growth factor (EGF), interleukin 1 (IL-1), interleukin-6 (IL-6), interleukin-10 (IL-10), interleukin-18 (IL-18), leptin, macrophage inflammatory protein 1-alpha (MIP-1I), myeloperoxidase, neurotrophin 3 (NT-3), plasminogen activator inhibitor-1 (PAI-1), Prolactin (PRL), RANTES, resistin, S100B protein, soluble tumor necrosis factor alpha receptor type 2 (sTNF-IRII), and tumor necrosis factor alpha (TNF-I). These biomarkers or any combination thereof can be used for MDD diagnosis, stratification of patients for clinical trials, and/or patient monitoring.

Example 2 - Using Proteomics to Analyze Multiplex Biomarkers

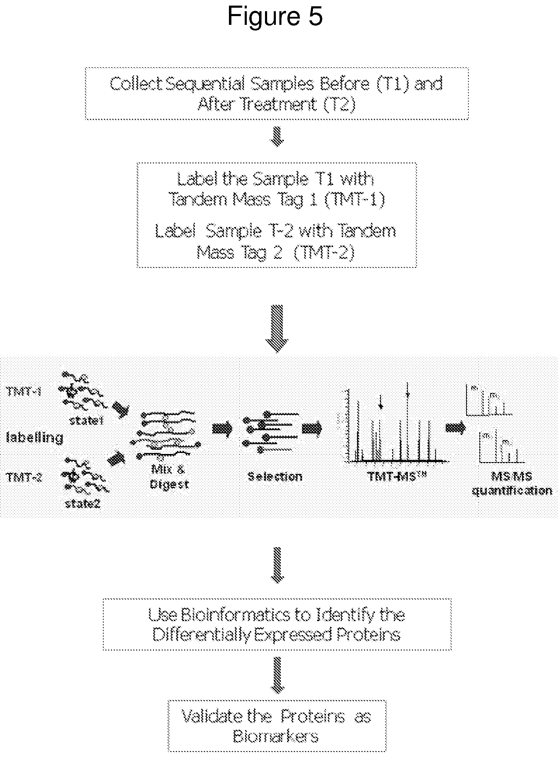

As shown in Figure 5, treatment-relevant biomarkers are identified using tandem mass spectrometry. Biological samples are collected pre- and post-treatment. The samples are labeled with different Tandem Mass Tags (TMT) and mixed for TMT-MS™ (Proteome Sciences, United Kingdom). Following fragmentation/digestion with a suitable enzyme (e.g., trypsin), TMT labeled fragments are selected for analysis by liquid chromatography MS/MS. The ratio of protein expression between samples is revealed by MS/MS by comparing the intensities of the individual reporter group signals. Bioinformatic analysis is used to determine the proteins that are differentially expressed. The identified proteins are then validated as potential biomarkers (e.g., using specific antibodies, and ELISA) over a defined period of time after treatment to establish a subset of pharmacodynamic biomarkers. Statistical analysis of a subject's changes in analyte expression levels is performed to correlate analytes with treatment efficacy. Upon statistical evaluation where statistical significance is defined as $p < 0.05$, biomarkers having a p value greater than 0.05 are selected as biomarkers associated with therapy-responsive MDD.

Example 3

Clinical results were obtained from serum samples from 50 MDD patients and 20 normal subjects. The serum levels of each of the markers (listed below) were determined by quantitative immunoassay.

A binary logistic regression optimization was used to fit the clinical data with selected markers in each group against the clinical results from the "gold standard" clinical evaluation. The result of the fit is a set of coefficients for the list of markers in the group. For example, A1AT (I1), A2M (I2), apolipoprotein CIII (I3), and TNF alpha (I4) were selected as the four markers representing the inflammatory group. Using binary logic regression against clinical results, four coefficients and the constants for these markers were calculated. The vector for the inflammatory group was constructed as follows:

$$V_{infla} = 1/(1+ \exp-(CI0 + CI1*I1 + CI2*I2+CI3*I3+CI4*I4)) \qquad (1)$$

Where CI0 = -7.34

CI1= -0.929

CI2=1.10

CI3=5.13

CI4=6.48

$V_{infla}$ represented the probability of whether a given patient had MDD using the measured inflammatory markers.

In the same way, vectors for other groups of markers were derived for MDD. Four markers were chosen to represent the metabolic group: M1=ASP, M2=prolactin, M3=resistin, and M4=testosterone. Using the same method of binary logistic regression described above for the clinical data, a set of coefficients and a vector summary were developed for patient metabolic response:

$$V_{meta} = 1/(1+ \exp -(Cm0+Cm1*M1+Cm2*M2+Cm3*M3+Cm4*M4)) \qquad (2)$$

Where Cm0 = -1.10
Cm1=0.313
Cm2=2.66
Cm3=0.82
Cm4=-1.87

$V_{meta}$ represented the probability of whether a given patient had MDD using the measured metabolic markers.

Two markers were chosen to represent the HPA group: H1=EGF and H2=G-CSF. Again, using the same method of binary logistic regression on the clinical data as above, a set of coefficients and a vector summary were developed for patient HPA response:

$$V_{hpa}= 1/(1+ \exp -(Ch0+Ch1*H1+Ch2*H2)) \qquad (3)$$

Where Ch0 = -1.87
Ch1=7.33
Ch2=0.53
$V_{hpa}$ represented the probability of whether a given patient has MDD using the measured HPA markers.

Using these three parameters, a hypermap representation of patients diagnosed with MDD and a normal subject control group was constructed and shown in Figure 3

Certain external factors, disease or therapeutics, can influence the expression of one or more biomarkers that are components of a vector within a hypermap. Figure 4 is a hypermap developed to demonstrate the response pattern for a series of MDD patients who initiated therapy with the antidepressant LEXAPRO™. Figure 4 shows changes in BHYPERMAP™ in a subset of Korean MDD patients after treatment with LEXAPRO™. Data for MDD patients at baseline are represented by filled circles. Data points after two to three weeks of treatment are represented by filled triangles, and data points after eight weeks of treatment are represented by open squares. Open circles represent data for normal subjects. This demonstrates that the technology can be used to define changes in an individual pattern in response to antidepressant therapy.

**Claims**

1. A method for identifying biomarkers of neuropsychiatric disease, comprising:

   (a) calculating a first diagnostic disease score for a subject having said neuropsychiatric disease, wherein said first diagnostic disease score is calculated prior to administration of transcranial magnetic stimulation to said subject;
   (b) measuring the levels of one or more analytes in a first biological sample obtained from said subject prior to administration of said transcranial magnetic stimulation, wherein the first biological sample is a blood, serum, or plasma sample;
   (c) calculating a second diagnostic disease score for said subject after administration of said transcranial magnetic stimulation;
   (d) measuring the levels of said one or more analytes in a second biological sample obtained from said subject after administration of said transcranial magnetic stimulation, wherein the second biological sample is a blood, serum, or plasma sample; and
   (e) identifying one or more analytes as being biomarkers for said neuropsychiatric disease, wherein said one or more analytes are identified as biomarkers if they are differentially expressed between said first and second biological samples, wherein said differential expression of said one or more analytes correlates to a positive or negative change in said subject's diagnostic score.

2. The method of claim 1, wherein the neuropsychiatric disease is major depressive disorder (MDD).

3. The method of claim 1, wherein said diagnostic scores are determined by clinical assessment.

4. The method of claim 1, wherein said administration of transcranial magnetic stimulation comprises repetitive transcranial magnetic stimulation or stimulating a prefrontal cortex of said subject.

5. The method of claim 1, wherein said second biological sample is collected from said subject hours, days, weeks, or months after administering trans cranial magnetic stimulation to said subject, preferably wherein steps (c), (d), and (e) of the method of claim 1 are repeated at intervals of time after administering trans cranial magnetic stimulation to said subject.

6. The method of claim 1, wherein said subject is monitored using molecular imaging technology.

7. The method of claim 1, wherein said subject receives one or more additional forms of therapeutic intervention to said subject, preferably wherein said one or more additional forms of therapeutic intervention are selected from the group consisting of cognitive behavioral therapy, drug therapy, therapeutic interventions that are behavioral in nature, group therapies, interpersonal therapies, psychodynamic therapies, relaxation or meditative therapies, and traditional psychotherapy.

8. The method of claim 1, further comprising:

   (f) using biomarker hypermapping technology to identify specific groups of analytes that are differentially expressed between said first and second biological samples, wherein said differential expression of a group of analytes correlates to a positive or negative change in said subject's hyperspace pattern.

9. A method for assessing a treatment response to maintain, adjust or stop said treatment in a mammal having major depressive disorder, comprising:

   (a) measuring the levels of inflammatory markers, HPA axis markers, and metabolic markers present in a first biological sample obtained from said mammal prior to administration of said treatment to obtain first numerical values for the levels of said markers, wherein the first biological sample is a blood, serum, or plasma sample, and wherein said inflammatory markers are alpha 1 antitrypsin, alpha 2 macroglobulin, apolipoprotein CIII, and tumor necrosis factor alpha, said HPA axis markers are epidermal growth factor and granulocyte colony stimulating factor, and said metabolic markers are acylation stimulating protein, prolactin, resistin, and testosterone;

(b) determining a first diagnostic disease score for said mammal, wherein said first diagnostic disease score is calculated using said first numerical values;

(c) measuring the levels of said inflammatory markers, said HPA axis markers, and said metabolic markers present in a second biological sample obtained from said mammal after administration of said treatment to obtain second numerical values for the levels of said markers, wherein the second biological sample is a blood, serum, or plasma sample;

(d) determining a second diagnostic disease score for said mammal, wherein said second diagnostic disease score is calculated using said second numerical values; and

(e) comparing said first diagnostic disease score to said second diagnostic disease score; wherein said method comprises using a hypermap that comprises using a score for said levels of said inflammatory markers, a score for said levels of said HPA axis markers, and a score for said levels of said metabolic markers to compare said first and second diagnostic disease scores.

10. The method of claim 9, wherein said mammal is a human.

11. The method of claim 9, wherein said treatment is transcranial magnetic stimulation.

12. The method of claim 9, wherein at least one of said first and second diagnostic disease score is calculated using numerical values for the levels of said inflammatory markers, said HPA axis markers, said metabolic markers, and at least two neurotrophic markers present in said first and/or second biological sample, wherein said at least two neurotrophic markers are selected from the group consisting of brain-derived neurotrophic factor, S100B, neurotrophin 3, reelin, glial cell line derived neurotrophic factor, and artemin.

13. The method of claim 9, further comprising repeating steps (c), (d), and (e) over time, and reinitiating, maintaining, adjusting, or stopping said treatment.

**Patentansprüche**

1. Ein Verfahren zur Identifizierung von Biomarker einer neuropsychiatrischen Erkrankung, umfassend:

(a) Berechnung eines ersten diagnostischen Erkrankungs-Scores für ein Subjekt mit jener neuropsychiatrischen Erkrankung, wobei jener erste diagnostische Erkrankungs-Score vor Verabreichung von transkranieller Magnetstimulation an jenes Subjekt berechnet wird;

(b) Messung der Spiegel von einem oder mehreren Analyten in einer ersten biologischen Probe, die von jenem Subjekt vor Verabreichung jener transktaniellen Magnetstimulation erhalten wurde, wobei die erste biologische Probe eine Blut-, Serum- oder Plasmaprobe ist;

(c) Berechnung eines zweiten diagnostischen Erkrankungs-Scores für jenes Subjekt nach Verabreichung jener transkraniellen Magnetstimulation;

(d) Messung der Spiegel jenes einen oder jener mehreren Analyten in einer zweiten biologischen Probe, die von jenem Subjekt nach Verabreichung jener transkraniellen Magnetstimulation erhalten wurde, wobei die zweite biologische Probe eine Blut-, Serum- oder Plasmaprobe ist; und

(e) Identifizierung eines oder mehrerer Analyten als Biomarker für jene neuropsychiatrische Erkrankung, wobei jener eine oder jene mehrere Analyten als Biomarker identifiziert werden, wenn sie in jener ersten und jener zweiten biologischen Probe unterschiedlich exprimiert sind, wobei jene unterschiedliche Expression jenes einen oder jener mehreren Analyten mit einer positiven oder negativen Veränderung im diagnostischen Score jenes Subjekt korreliert.

2. Das Verfahren von Anspruch 1, wobei die neuropsychiatrische Erkrankung eine schwere Depression (MDD) ist.

3. Das Verfahren von Anspruch 1, wobei jene diagnostischen Scores durch klinische Beurteilung bestimmt werden.

4. Das Verfahren von Anspruch 1, wobei jene Verabreichung von tzanskranieller Magnetstimulation wiederholte transkranielle Magnetstimulation oder Stimulieren eines präfrontalen Cortex jenes Subjekts umfasst.

**5.** Das Verfahren von Anspruch 1, wobei jene zweite biologische Probe Stunden, Tage, Wochen oder Monate nach Verabreichung transkranieller Magnetstimulation an jenes Subjekt von jenem Subjekt gewonnen wird, wobei die Schritte (c), (d) und (e) des Verfahrens von Anspruch 1 vorzugsweise in Zeitintervallen nach Verabreichung transkranieller Magnetstimulation an jenes Subjekt wiederholt werden.

**6.** Das Verfahren von Anspruch 1 wobei jenes Subjekt unter Verwendung von molekularer Bildgebungs-Technologie überwacht wird.

**7.** Das Verfahren von Anspruch 1, wobei jenes Subjekt eine oder mehrere zusätzliche Formen von therapeutischer Intervention bei jenem Subjekt erhält,
wobei jene eine oder jene mehreren zusätzlichen Formen von therapeutischer Intervention vorzugsweise ausgewählt sind aus der Gruppe bestehend aus kognitiver Verhaltenstherapie, medikamentöser Therapie, therapeutischen Interventionen, die in ihrem Wesen verhaltensbezogen sind, Gruppentherapien, interpersonellen Therapien, psychodynamischen Therapien, Entspannungs- oder meditativen Therapien und traditioneller Psychotherapie.

**8.** Das Verfahren von Anspruch 1, außerdem umfassend:

(f) Verwendung von Biomarker-Hypermapping-Technologie, um spezifische Gruppen von Analyten zu identifizieren, die in der ersten und der zweiten biologischen Probe unterschiedlich exprimiert sind, wobei jene unterschiedliche Expression einer Gruppe von Analyten mit einer positiven oder negativen Veränderung im Hyperspace-Muster jenes Subjekts korreliert.

**9.** Ein Verfahren zur Beurteilung eines Hehandlungserfolgs, um jene Behandlung in einem Säugetier mit einer schweren Depression beizubehalten, anzupassen oder zu beenden, umfassend:

(a) Messung der Spiegel von inflammatorischen Markern, HPA-Achsen-Marken und metabolischen Markern, die in einer ersten biologischen Probe vorhanden sind, die von jenem Säugetier vor Verabreichung jener Behandlung erhalten wurde, um erste numerische Werte für die Spiegel jener Marker zu erhalten, wobei die erste biologische Probe eine Blut-, Serum- oder Plasmaprobe ist und wobei jene inflammatorischen Marker alpha-1-Antitrypsin, alpha-2-Makroglobulin, Apolipoprotein CIII und Tumornekrosefaktor-alpha sind, jene HPA-Achsen-Marker epidermaler Wachstumsfaktor und Granulozyten-Kolonie-stimulierender Faktor sind und jene metabolischen Marker Acylierung-stimulierendes Protein, Prolaktin, Resistin und Testosteron sind;
(b) Bestimmung eines ersten diagnostischen Erkrankungs-Scores für jenes Säugetier, wobei jener erste diagnostische Erkrankungs-Score unter Verwendung jener ersten numerischen Werte berechnet wird;
(c) Messung der Spiegel jener inflammatorischen Marker, jener HPA-Achsen-Marker und jener metabolischen Marker, die in einer zweiten biologischen Probe vorhanden sind, die von jenem Säugetier nach Verabreichung jener Behandlung erhalten wurde, um zweite numerische Werte für die Spiegel jener Marker zu erhalten, wobei die zweite biologische Probe eine Blut-, Serum- oder Plasmaprobe ist;
(d) Bestimmung eines zweiten diagnostischen Erkrankungs-Scores für jenes Säugetier, wobei jener zweite diagnostische Erkrankungs-Score unter Verwendung jener zweiten numerischen Werte berechnet wird; und
(e) Vergleich jenes ersten diagnostischen Erkrankungs-Scores mit jenem zweiten diagnostischen Erkrankungs-Score; wobei jenes Verfahren die Verwendung einer Hypermap umfasst, die die Verwendung eines Scores für jene Spiegel jener inflammatorischen Marker, eines Scores für jene Spiegel jener HPA-Achsen-Marker und eines Scores für jene Spiegel jener metabolischen Marker umfasst, um jenen ersten und jenen zweiten diagnostischen Erkrankungs-Score zu vergleichen.

**10.** Das Verfahren von Anspruch 9, wobei jenes Säugetier ein Mensch ist.

**11.** Das Verfahren von Anspruch 9, wobei jene Behandlung transkranielle Magnetstimulation ist.

**12.** Das Verfahren von Anspruch 9, wobei mindestens einer von jenem ersten und jenem zweiten diagnostischen Score unter Verwendung von numerischen Werten für die Spiegel jener inflammatorischen Marker, jener HPA-Achsen-Marker, jener metabolischen Marker und mindestens zweier neurotropher Marker, die in jener ersten und/oder jener zweiten biologischen Probe vorhanden sind, berechnet wird, wobei jene mindestens zwei neurotrophen Marker ausgewählt sind aus der Gruppe bestehen aus *Biain-derived-nerootrophic-Factor,* S100B, Neurotrophin-3, Reelin, *Glial cell line-derived neurotrophic factor* und Artemin.

**13.** Das Verfahren von Anspruch 9, außerdem umfassend die Wiederholung der Schritte (c), (d) und (e) im Laufe der Zeit und das Wiederaufnehmen, Beibehalten, Anpassen oder Beenden jener Behandlung.


**Revendications**

1.  Procédé d'identification de biomarqueurs d'une maladie neuropsychiatrique, comprenant :

    (a) le calcul d'un premier score pathologique de diagnostic pour un sujet souffrant de ladite maladie neuropsychiatrique, dans lequel ledit premier score pathologique de diagnostic est calculé avant l'administration de la stimulation magnétique transcrânienne audit sujet ;
    (b) la mesure des taux d'un ou de plusieurs analytes dans un premier échantillon biologique obtenu dudit sujet avant l'administration de ladite stimulation magnétique transcrânienne, dans laquelle le premier échantillon biologique est un échantillon de sang, de sérum, ou de plasma ;
    (c) le calcul d'un second score pathologique de diagnostic pour ledit sujet après l'administration de ladite stimulation magnétique transcrânienne ;
    (d) la mesure des taux desdits un ou plusieurs analytes dans un second échantillon biologique obtenu dudit sujet après l'administration de ladite stimulation magnétique transcrânienne, dans laquelle le second échantillon biologique est un échantillon de sang, de sérum, ou de plasma ; et
    (e) l'identification d'un ou de plusieurs analytes comme étant des biomarqueurs pour ladite maladie neuropsychiatrique, dans laquelle lesdits un ou plusieurs analytes sont identifiés comme des biomarqueurs s'ils sont exprimés de façon différentielle entre lesdits premier et second échantillons biologiques, dans laquelle ladite expression différentielle desdits un ou plusieurs analytes corrèle avec une variation positive ou négative du score de diagnostic dudit sujet.

2.  Procédé selon la revendication 1, dans lequel la maladie neuropsychiatrique est un trouble dépressif majeur (TDM).

3.  Procédé selon la revendication 1, dans lequel lesdits scores de diagnostic sont déterminés par une évaluation clinique.

4.  Procédé selon la revendication 1, dans lequel ladite administration de stimulation magnétique transcrânienne comprend une stimulation magnétique transcrânienne répétitive ou la stimulation du cortex préfrontal dudit sujet.

5.  Procédé selon la revendication 1, dans lequel ledit second échantillon biologique est prélevé chez ledit sujet des heures, des jours, des semaines, ou des mois après l'administration de la stimulation magnétique transcrânienne audit sujet,
    de préférence dans lequel les étapes (c), (d), et (e) du procédé selon la revendication 1 sont répétées à des intervalles de temps après l'administration de la stimulation magnétique transcrânienne audit sujet.

6.  Procédé selon la revendication 1, dans lequel ledit sujet est suivi en utilisant la technologie de l'imagerie moléculaire.

7.  Procédé selon la revendication 1, dans lequel ledit sujet reçoit une ou plusieurs formes supplémentaires d'intervention thérapeutique audit sujet,
    de préférence dans lequel lesdites une ou plusieurs formes supplémentaires d'intervention thérapeutique sont choisies dans le groupe constitué de la thérapie cognitive et comportementale, la pharmacothérapie, les interventions thérapeutiques qui sont de nature comportementales, les thérapies de groupe, les thérapies interpersonnelles, les thérapies psychodynamiques, les thérapies par relaxation ou méditation, et la psychothérapie traditionnelle.

8.  Procédé selon la revendication 1, comprenant en outre :

    (f) l'utilisation de la technologie de l'hypercartographie des biomarqueurs pour identifier des groupes spécifiques d'analytes qui sont exprimés de façon différentielle entre lesdits premier et second échantillons biologiques, dans lequel ladite expression différentielle d'un groupe d'analytes corrèle avec une variation positive ou négative du profil d'hyperespace dudit sujet.

9.  Procédé d'évaluation d'une réponse à un traitement pour maintenir, ajuster ou arrêter ledit traitement chez un mammifère souffrant de trouble dépressif majeur, comprenant :

    (a) la mesure des taux des marqueurs inflammatoires, des marqueurs de l'axe HHS, et des marqueurs métaboliques présents dans un premier échantillon biologique obtenu dudit mammifère avant l'administration dudit traitement pour obtenir des premières valeurs numériques pour les taux desdits marqueurs, dans laquelle le premier échantillon biologique est un échantillon de sang, de sérum, ou de plasma, et dans laquelle lesdits marqueurs inflammatoires sont l'alpha-1-antitrypsine, l'alpha-2-macroglobuline, l'apolipoprotéine CIII, et le facteur de nécrose tumorale alpha, lesdits marqueurs de l'axe HHS sont le facteur de croissance épidermique et le facteur de stimulation des colonies de granulocytes, et lesdits marqueurs métaboliques sont la protéine de stimulation de l'acylation, la prolactine, la résistine, et la testostérone ;

(b) la détermination d'un premier score pathologique de diagnostic pour ledit mammifère, dans laquelle ledit premier score pathologique de diagnostic est calculé en utilisant lesdites premières valeurs numériques ;

(c) la mesure des taux desdits marqueurs inflammatoires, desdits marqueurs de l'axe HHS, et desdits marqueurs métaboliques présents dans un second échantillon biologique obtenu dudit mammifère après l'administration dudit traitement pour obtenir des secondes valeurs numériques pour les taux desdits marqueurs, dans laquelle le second échantillon biologique est un échantillon de sang, de sérum, ou de plasma ;

(d) la détermination d'un second score pathologique de diagnostic pour ledit mammifère, dans laquelle ledit second score pathologique de diagnostic est calculé en utilisant lesdites secondes valeurs numériques ; et

(e) la comparaison dudit premier score pathologique de diagnostic audit second score pathologique de diagnostic ; dans laquelle ledit procédé comprend l'utilisation d'une hypercarte qui comprend l'utilisation d'un score pour lesdits taux desdits marqueurs inflammatoires, d'un score pour lesdits taux desdits marqueurs de l'axe HHS, et d'un score pour lesdits taux desdits marqueurs métaboliques pour comparer lesdits premier et second scores pathologiques de diagnostic.

10. Procédé selon la revendication 9, dans lequel ledit mammifère est un être humain.

11. Procédé selon la revendication 9, dans lequel ledit traitement est la stimulation magnétique transcrânienne.

12. Procédé selon la revendication 9, dans lequel au moins l'un desdits premier et second scores pathologiques de diagnostic est calculé en utilisant des valeurs numériques pour les taux desdits marqueurs inflammatoires, desdits marqueurs de l'axe HHS, desdits marqueurs métaboliques, et d'au moins deux marqueurs neurotrophiques présents dans ledit premier et/ou second échantillon biologique, dans lequel lesdits au moins deux marqueurs neurotrophiques sont choisis dans le groupe constitué du facteur neurotrophique dérivé du cerveau, S100B, la neurotrophine 3, la rééline, le facteur neurotrophe dérivé de la glie, et l'artémine.

13. Procédé selon la revendication 9, comprenant en outre la répétition des étapes (c), (d), et (e) dans le temps, et la réinitialisation, le maintien, l'ajustement, ou l'arrêt dudit traitement.

# Figure 1

```
┌─────────────────────────────────────────┐
│         Select patient population        │
│          and control subjects            │
└─────────────────────────────────────────┘
                    │
                    ▼
┌─────────────────────────────────────────┐
│  Employ a large Biomarker Library (~200 mkrs) │
│   Add Disease Relevant Content (optional)     │
└─────────────────────────────────────────┘
                    │
                    ▼
┌─────────────────────────────────────────┐
│        Measure biomarker expression       │
└─────────────────────────────────────────┘
                    │
                    ▼
┌─────────────────────────────────────────┐
│    Perform Univariate Statistical Analysis    │
│    Select Significant Analytes (P< 0.05)      │
└─────────────────────────────────────────┘
                    │
                    ▼
┌─────────────────────────────────────────┐
│    Perform Clustering to Optimize and/or      │
│     Reduce the Number of Markers              │
└─────────────────────────────────────────┘
                    │
                    ▼
┌─────────────────────────────────────────┐
│  Use Biological Understanding e.g. Pathway    │
│    Analysis to Finalize Biomarkers            │
└─────────────────────────────────────────┘
```

# Figure 2

Begin with an
Established Collection of
Biomarkers

Establish a Cohort of MDD
Patients by use of a "Gold
Standard" Clinical Interview

Obtain Plasma or Serum
Samples from the Patients Prior
to Treatment

Treat Patients With TMS or a
Mock Treatment

Obtain Plasma or Serum
Samples from Patients
After Treatment

Identify Patients with a
Positive Clinical Response

Measure Biomarker
Expression Levels

Identify Biomarkers whose Expression
Correlates with Positive Treatment
Response

Figure 3

Figure 4

# Figure 5

Figure 6

```
┌──────────────────────────────┐        ┌──────────────────────────────┐
│   Biomarker Library Database  │        │      Patient Database         │
│     Storing Different Sets of │        │ (measured values of individual│
│   Combinations of Biomarkers  │        │          biomarkers)          │
│   and Associated Coefficients │        │             720               │
│      for Each Combination     │        │                               │
│             710               │        │                               │
└──────────────────────────────┘        └──────────────────────────────┘
                    \                          /
                     \                        /
                    ┌──────────────────────────────┐
                    │ Diagnostic Processing Engine  │
                    │             730               │
                    └──────────────────────────────┘
                                  │
                                  │
                    ┌──────────────────────────────┐
                    │      Diagnostic Output        │
                    │             740               │
                    └──────────────────────────────┘
```

EP 2 414 824 B1

Figure 7

800

820

Memory

840

Input/Output
Devices

Input/Output

850

Processor
810

Storage Device
930

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61165662 A
- WO 02057790 A
- US 20080281531 A
- EP 1586657 A
- KR 20050115436

- US 910217 P
- US 60824471 B
- US 850550 A
- WO 2007067819 A

### Non-patent literature cited in the description

- **IKEDA et al.** *Biochem. Biophys. Res. Comm.,* 2005, vol. 327, 218-224
- **PAUS ; BARRETT.** *J. Psychiatry Neurosci.,* 2004, vol. 29, 268-79

- **MICHAEL TAYLOR ; MAX FINK.** Melancholia: The Diagnosis, Pathophysiology, and Treatment or Repressive Illness. University Press, 2006, 91-92
- **MAES.** Inflammatory Response System (IRS) model of depression. *Adv. Exp. Med. Biol.,* 1999, vol. 461, 25-46